# EUROPEAN PATENT APPLICATION

(11) **EP 2 559 431 A1**
(43) Date of publication of application: **20.02.2013**
(21) Application number: 11177863.5
(22) Date of filing: 17.08.2011
(51) Int. Cl.: A61K 9/20, A61K 31/44

(54) **Pharmaceutical composition comprising 4-[4-[[4-chloro-3-(trifluoromethyl)phenyl]carbamoylamino]phenoxy]-N-methyl-pyridine-2-carboxamide**

(71) Applicant: Ratiopharm GmbH, 89079 Ulm (DE)
(72) Inventor: Janssen, Christian, 89171 Illerkirchberg (DE); Stefan, Ralph, 88370 Ebenweiler (DE)
(74) Representative: Teipel, Stephan

(57) **Abstract**

The present invention relates to a pharmaceutical composition having a density of more than 1.55g/cm³ in the form a tablet comprising 4-[4-[[4-chloro-3-(tifluoromethyl)phenyl]carbamoylamino]phenoxy]-N-methyl-pyridine-2-carboxamide or a pharmaceutically acceptable salt thereof as active agent and at least one pharmaceutically acceptable excipient as well as a method of manufacturing such pharmaceutical composition.

## Description

The present invention relates to a pharmaceutical composition in the form a tablet comprising 4-[4-[[4-chloro-3-(tifluoromethyl)phenyl]carbamoylamino]phenoxy]-N-methyl-pyridine-2-carboxamide or a pharmaceutically acceptable salt thereof as active agent and at least one pharmaceutically acceptable excipient as well as a method of manufacturing such pharmaceutical composition.

4-[4-[[4-Chloro-3-(trifluoromethyl)phenyl]carbamoylamino]phenoxy]-N-methyl-pyridine-2-carboxamide, also known as Sorafenib, has the following chemical structure:

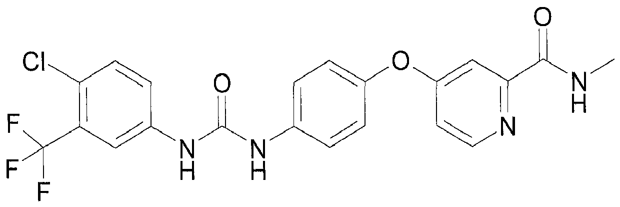

Sorafenib is reported to be a small molecular inhibitor of several protein kinases, including RAF, VEGFR-2PK30, and PDGFR kinases. These enzymes are all molecular targets of interest for the treatment of hyper-proliferative diseases, including cancer.

ω-Carboxylaryl substituted diphenyl ureas including Sorafenib and its synthesis are apparently disclosed in WO 2000/42012. This document also describes various pharmaceutically acceptable salts of the compounds.

WO 2006/026501 discloses pharmaceutical compositions comprising a solid dispersion of Sorafenib.

WO 2006/034796 discloses a process for preparing Sorafenib and its tosylate salt. The tosylate salt is said to be obtained in crystalline form.

WO 2006/034797 describes polymorphic forms of Sorafenib tosylate designated polymorph I and polymorph III, whereas the polymorph described in WO 00/42012 is designated polymorph II. It also describes a monomethanol solvate and a monoethanol solvate.

Typically Sorafenib is administered orally, as this route provides comfort and convenience of dosing. In order to support a high compliance the oral pharmaceutical composition should not have to be taken in more than three times a day, the less the better, and in the case of a tablet the dimensions of the tablet should not be too large to allow a good swallowing. Therefore, WO 2006/094626 suggests a pharmaceutical composition containing a high-drug-load of Sorafenib.

However, tablets containing a high drug load may be difficult to prepare, have disadvantages in their mechanical stability and dissolution properties. Therefore, there is still a need for improved pharmaceutical compositions comprising Sorafenib which should be applied not more than three times a day in order to achieve an effective plasma level of the active agent, which should not be too large to provide good swallowing, which exhibit good release properties, bioavailability of the active agent, chemical and mechanical stability and a good industrial applicability of the manufacturing process thereof. Moreover, a pharmaceutical composition comprising Sorafenib as active agent should have low inter-patient variability, good overall therapeutic efficacy, excellent flow properties and good compressability of the composition during manufacturing, a good dissolution profile making the pharmaceutical composition suitable as immediate release composition, and low hygroscopicity and electrostatic charging during manufacture and storage.

It has now surprisingly been found that the above problems can be solved by providing a pharmaceutical composition in form of a tablet comprising Sorafenib, wherein the tablet core has a higher density as usual. This allows a relatively low proportion of the active agent in the tablet core and at the same time an amount of the active agent being sufficient for a single dosage form and nevertheless resulting in a tablet having dimensions allowing the tablet to be easily swallowed. It has been surprisingly found that despite the high density of the tablet core the dissolution profile of the tablet remains suitable for an immediate release formulation. Moreover, it has surprisingly been found that Sorafenib can be admixed with high density excipients and that despite the high differences in the densities of Sorafenib and the excipients nevertheless pharmaceutical preparations are obtained having a good content uniformity. This finding is particularly unexpected because mixtures containing powders of different densities tend to demix in which case it is difficult to prepare tablets having the required high content uniformity.

Thus, the present invention relates to a pharmaceutical composition in the form of a tablet, comprising 4-[4-[[4-chloro-3-(trifluoromethyl)phenyl]carbamoylamino]phenoxy]-N-methylpyridine-2-carboxamide or a pharmaceutically acceptable salt thereof as active agent and at least one pharmaceutically acceptable excipient, characterized in that the tablet core has density of above 1.55 g/cm³.

The density of the tablet core preferably is above 1.60 g/cm³, more preferably above 1.65 g/cm³ and most preferably above 1.75 g/cm³.

The pharmaceutical composition of the present invention may comprise Sorafenib or any pharmaceutically acceptable salt thereof, including solvates, hydrates and any polymorphs thereof. Suitable pharmaceutically acceptable salts are well known to those skilled in the art and include salts of inorganic or organic acids, such as hydrochloride acid, hydrobromic acid, sulfuric acid, phosphoric acid, methanesulfonic acid, trifluoromethanesulfonic acid, benzenesulfonic acid, p-toluenesulfonic acid, naphthalenesulfonic acid, acetic acid, malic acid, tartaric acid, citric acid, lactic acid, oxalic acid, succinic acid, fumaric acid, maleic acid, benzoic acid, salicylic acid, phenylacetic acid and mandelic acid. Preferably the pharmaceutical composition of the present invention comprises Sorafenib in the form of its pharmaceutically acceptable salt, in particular as tosylate or besylate salt, most preferably in the form of the tosylate salt in polymorphic form III as described in WO 2006/034797.

One of the advantages of the pharmaceutical composition of the present invention is that the size of the tablet can be low although at the same time the content of active agent is also rather low. For example, the pharmaceutical composition can comprise the active agent in an amount of 25 to less than 55 % by weight of the total weight of the tablet core. Preferably, the pharmaceutical composition comprises the active agent in an amount of more than 45 to less than 55 % by weight of the total weight of the tablet core.

In one embodiment the pharmaceutical composition of the present invention may contain the active agent in micronized form. Micronization can be achieved by standard milling methods known to a skilled person. The micronized form can have a mean particle size of from 0.5 µm to 10 µm, preferably from 1 µm to 7 µm, more preferably from 2 µm to 5 µm. The indicated particle size is the mean d 0.5 of the particle size distribution measured by laser diffraction on a Malvern Mastersizer 2000 in liquid light paraffin with a obscuration rate of 10-30%, a stirrer speed of 2500 upm, and sonication at 50% for 15 min.

The pharmaceutical composition of the present invention comprises one or more pharmaceutically acceptable excipients, such as fillers, binding agents, lubricants, flow enhancers, anti-sticking agents, disintegrating agents and solubilizers. As pharmaceutically acceptable excipients conventional excipients known to a person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Sixth Edition, American Pharmaceutical Association, Washington, USA, and Pharmaceutical Press, London.

Preferred examples of fillers are lactose, mannitol, sorbitol, microcrystalline cellulose and in particular dibasic calcium phosphate. The filler is suitably present in an amount of 0 to 75 % by weight, preferably of 20 to 40 % by weight of the total weight of the tablet core.

The binding agent can be microcrystalline cellulose (MCC), silicified microcrystalline cellulose or hydroxypropylmethyl cellulose (HPMC, Hypromellose). The binding agent is suitably present in an amount of 5 to 20 % by weight, preferably of 2 to 15 % by weight of the total weight of the tablet core.

The lubricant is preferably a stearate, more preferably an earth alkali metal stearate, such as magnesium stearate. The lubricant is suitably present in an amount of 0.1 to 2 % by weight, preferably of 0.5 to 1 % by weight of the total weight of the tablet core.

Preferred disintegrating agents are croscarmellose sodium, sodium carboxymethyl starch and cross-linked polyvinylpyrrolidone (crospovidone). The disintegrating agent is suitably present in an amount of 0.1 to 20 % by weight, more preferably of 3 to 10 % by weight of the total weight of the tablet core.

The flow enhancer can be colloidal silicon dioxide. The flow enhancer is suitably present in an amount of 0.5 to 8 % by weight, more preferably of 0.5 to 3 % by weight of the total weight of the tablet core.

The anti-sticking agent is for example talcum and may be present in an amount of 1 to 5 % by weight, preferably of 1.5 to 3 % by weight of the total weight of the tablet core.

If desired, an improvement of the solubility of the active pharmaceutical ingredient can be achieved by the addition of complex forming agents/compounds (e.g. sodium benzoate, sodium salicylate or cyclodextrins), alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®), polyoxypropylene polyoxyethylene copolymers (poloxamer; e.g. Pluronic®) or sodium dodecyl sulfate and may be present in amounts of 0.1 to 7 % by weight, preferably of 0.2 to 1 % by weight of the total weight of the tablet core.

Alternatively, a pseudo-emulsifier can be used. Its mechanism of action mainly relies on an enhancement of viscosity. However, pseudo-emulsifiers also possess emulsifying properties. Preferred pseudo-emulsifiers are for example cellulose ethers, gum Arabic or tragacanth and may be present in an amount of 1 to 10 % by weight, preferably of 3 to 7 % by weight of the total weight of the tablet core.

In one embodiment the pharmaceutical composition of the present invention comprises 20 to 40 % by weight of filler, 5 to 20 % by weight of binder, 3 to 10 % by weight of disintegrant, 0.2 to 1 % by weight of surfactant and 0.5 to 1 % of lubricant, each relating to the total weight of the tablet core. For example, the pharmaceutical composition may comprise about 50.1 % by weight of Sorafenib tosylate, preferably in polymorphic form III, more preferably micronized, about 28.3 % by weight of filler, in particular dibasic calcium phosphate, about 6.4 % by weight of disintegrant, in particular cross-linked polyvinylpyrrolidone, about 1.8 % by weight of a first binder, in particular Hypromellose, about 12.4 % by weight of a second binder, in particular silicified microcrystalline cellulose, about 0.3 % by weight of surfactant, in particular sodium dodecyl sulfate, and about 0.7 % by weight of lubricant, in particular magnesium stearate, each of the amounts relating to the total weight of the tablet core.

In order to ensure the required high density of the tablet core of the pharmaceutical composition of the present invention it is advantageous to select the excipients such, that they have a high density, in particular a density being higher than the density of the active agent. The densities of the ingredients before compression into a tablet are their bulk densities being measured according to usual methods known to a person skilled in the art.

Examples for pharmaceutically acceptable excipients with high density are calcium hydrogen phosphate (DiCafos AN, 2.89 g/cm³), calcium hydrogen phosphate dihydrate (DiCafos Dihydrat, 2.39 g/cm³), calcium phosphate (TriCafos, 3.14 g/cm³), calcium silicate (2.10 g/cm³), calcium sulphate (2.31 g/cm³), aluminium hydroxide (2.42 g/cm³) and/or calcium carbonate (2.73 g/cm³) ("Handbook of Pharmaceutical Excipients", Sixth Edition, American Pharmaceutical Association, Washington, USA, and Pharmaceutical Press, London and/or GESTIS database of hazardous substances from the German Social Accident Insurance).

Examples for other usual fillers with low density are microcrystalline cellulose (e.g. Avicel PH 102, 1.42-1.46 g/cm³), mannitol (1.51 g/cm³) and lactose monohydrate (1.545 g/cm³) ("Handbook of Pharmaceutical Excipients", Sixth Edition American Pharmaceutical Association, Washington, USA, and Pharmaceutical Press, London).

In order to allow good swallowability of the tablet and, thus, good patient compliance, it is preferable that the tablet has a low size. Therefore, in one embodiment of the present invention the tablet has a longest diameter below 13 mm, preferably below 12 mm, such as, for example, about 11 mm.

The tablet according to the invention shows for example a hardness of more than 50 N, preferably in the range of 60 to 150 N.

The pharmaceutical composition according to the invention shows good release properties. Furthermore, preference is given to administration forms wherein the active agent is delivered in a rapid manner also known as "immediate release" administration form. According to the present invention immediate release administration forms show a release profile wherein at least 75 % of the active agent is released after 30 minutes when measured with USP method, device 2 (paddle, 75 rpm, in 0.1 M HCl + 1 % sodium dodecyl sulfate).

The pharmaceutical composition according to the invention is stable for more than 18 months.

The pharmaceutical composition of the present invention may contain dosage amounts in the range of for example 100 to 400 mg of the active agent. For example, one tablet according to the invention may contain 100, 200, or 400 mg of the active agent, each calculated as Sorafenib in its free base form. For example, a tablet containing 274 mg Sorafenib tosylate contains 200 mg Sorafenib in its free base form.

The pharmaceutical compositions of the present invention can be manufactured according to standard methods known in the art. For example granules can be obtained by dry compaction or wet granulation. These granules can subsequently be mixed with the remaining excipients and can be compressed into tablets of suitable size. Tablets can also be obtained by direct compression of a suitable powder mixture, i.e. without any preceding granulation of the excipients. Suitable powder or granulate mixtures are also obtainable by spray drying, lyophilization, melt extrusion, pellet layering, coating of the active pharmaceutical ingredient or any other suitable method.

The above mentioned methods known in the art also include grinding and sieving techniques permitting the adjustment of the desired particle size distributions.

Thus, the present invention also relates to a method of manufacturing the above described pharmaceutical composition, wherein the active agent is blended with at least one pharmaceutically acceptable excipient and the resulting mixture is formulated into a tablet. This method may comprise a dry compaction or wet granulation step. Alternatively this method may comprise a direct compression step. Furthermore, the method may comprise a coating step.

The present invention will now be described by the following example which is not intended as being limiting.

### Example

| **Compound** | **[mg]** | **[%]** |
|---|---|---|
| Sorafenib tosylate form III, micronised | 282.81 | 50.05 |
| Dibasic calcium phosphate anhydrous (DiCafos AN) | 160.00 | 28.31 |
| Cross-linked polyvinyl-pyrrolidone (Kollidon CL) | 36.40 | 6.44 |
| Hypromellose (Methocel E5) | 10.20 | 1.80 |
| Silicified microcrystalline cellulose (Prosolv SMCC 90) | 70.00 | 12.39 |
| Sodium dodecyl sulfate | 1.70 | 0.30 |
| Magnesium stearate | 4.00 | 0.71 |
| **TOTAL** | **565.11** | **100** |

The active ingredient, DiCafos AN, Kollidon CL, Methocel E5 and 20 mg of the Prosolv SMCC 90 were mixed at 250 rmp for 2 min and then granulated with a 1% aqueous solution of sodium dodecyl sulfate. The granulate was dried at 40 °C in a drying oven and passed through a sieve (1000 µm). The residual amount of Prosolv SMCC 90 was added through a sieve (800 µm) and the resulting mixture was mixed for 10 min. The magnesium stearate was added through a sieve (800 µm) and the resulting mixture was mixed for another 3 min.

The final mixture was pressed to obtain biconvex tablets (11 mm Ø, hardness 60-150 N, density 1.77 g/cm³) on a Riva Piccola rotary tablet press.

The density was determined on a Micrometrics AccuPcy II 1340 V1.05 in a chamber of 3.5 cm³ using Helium as analysis gas at a temperature of 25 °C. Each sample of a single tablet was analysed ten times and the arithmetic mean was determined.

The dissolution rate of tablets according to the present invention was determined using the following equipment and measurement parameters:

### Dissolution (USP II)

**Equipment:** Dissolution bath AT 7 Smart, Sotax; Pump CY 7-50, Sotax; UV-Vis Spectral photometer 8453, On-line Multicell, Agilent; 1 mm cuvettes; DosaPrep® X8, Dosatec.

### Dissolution Parameters

| | |
|---|---|
| Dosage: | 200 mg Sorafenib, 1 tablet per vessel |
| Dissolution medium: | 0.1 N HCl, pH 1.2 + 0.5% SDS |
| Volume: | 900 ml |
| Temperature: | 37°C ± 0.5°C |
| Agitation: | paddle, 75 rpm |
| Sampling interval: | 4 x 5 minutes (0 - 20min) |
| | 1 x 10 minutes (20 - 30min) |
| | 2 x 15 minutes (30 - 60min) |
| | 1 x 30 minutes (60 - 90min), 150rpm |
| Total time: | 90 minutes |
| Blank: | 0.1 N HCl, pH 1.2 + 0.5% SDS |
| Sinker: | no |
| Filter: | 1 µm Glass fibre membrane |
| Measurement: | on-line UV/VIS measurement |
| Path length: | 1 mm |
| Wavelength: | 265 nm |
| Background: | subtract average over range, 400 to 450 nm. |

The dissolution rate was determined initially after preparation of the tablets, after 4 weeks storage at 25°C and 60% relative humidity, after 4 weeks storage at 40°C and 75% relative humidity, after 12 weeks storage at 30°C and 65% relative humidity and after 12 weeks storage at 40°C and 65% relative humidity. The results are summarized in the following table:

| | | | **Initial** | **4 weeks -25°C/60%** | **4 weeks -40°C/75%** | **12 weeks -30°C/65%** | **12 weeks -40°C/75%** |
|---|---|---|---|---|---|---|---|
| **Description** | | | tablets, white | tablets, white | tablets, white | tablets, white | tablets, white |
| **Dissolution** | | n=3 | | | | | |
| | 5 min [%] | | 41.9 ± 1.1 | 37.8 ± 0.8 | 40.8 ± 2.5 | 38.8 ± 1.5 | 43.2 ± 5.3 |
| | 10 min [%] | | 74.9 ± 2.9 | 70.7 ± 1.5 | 67.7 ± 2.3 | 69.0 ± 0.5 | 70.6 ± 5.3 |
| | 15 min [%] | | 90.2 ± 2.8 | 85.7 ± 1.9 | 80.1 ± 2.9 | 83.5 ± 1.4 | 82.4 ± 5.1 |

The data demonstrate that the tablets of the invention exhibit an immediate release profile both, initially as well as after storage for several weeks even under increased temperature and high humidity. Thus, the tablets have an excellent storage stability.

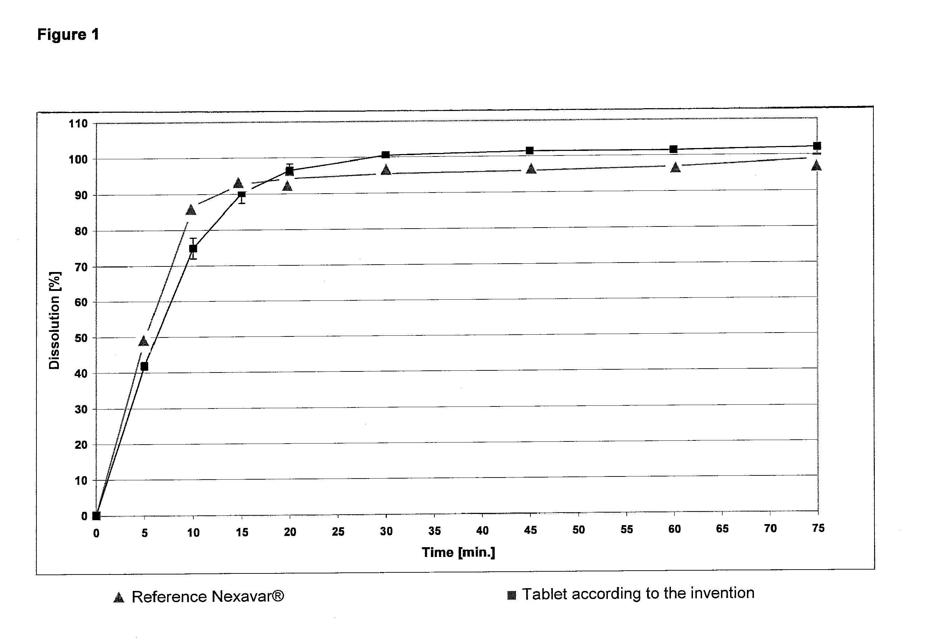

Furthermore, the dissolution profile of the tablet according to the invention as well as the dissolution profile of a commercial Nexavar^{®} 200 mg tablet as reference are shown in attached Figure 1.

## Claims

1. A pharmaceutical composition in the form a tablet comprising 4-[4-[[4-chloro-3-(tifluoromethyl)phenyl]carbamoylamino]phenoxy]-N-methyl-pyridine-2-carboxamide or a pharmaceutically acceptable salt thereof as active agent and at least one pharmaceutically acceptable excipient, **characterized in that** the tablet core has a density of above 1.55 g/cm³.

2. The pharmaceutical composition of claim 1, wherein the density is above 1.60 g/cm³, preferably above 1.65 g/cm³, more preferably above 1.75 g/cm³.

3. The pharmaceutical composition of claims 1 or 2, wherein the pharmaceutically acceptable salt is the tosylate or besylate salt, preferably the tosylate salt in polymorphic form III.

4. The pharmaceutical composition of any of the preceding claims, comprising the active agent in an amount of 25 to less than 55 % by weight of the total weight of the tablet core.

5. The pharmaceutical composition of claims 4, comprising the active agent in an amount of more than 45 to less than 55 % by weight of the total weight of the tablet core.

6. The pharmaceutical composition of any of the preceding claims, wherein the active agent is micronized.

7. The pharmaceutical composition of any of the preceding claims, further comprising 20 to 40 % by weight of filler, 5 to 20 % by weight of binder, 3 to 10 % by weight of disintegrant, 0.2 to 1 % by weight of surfactant and 0.5 to 1 % by weight of lubricant, each relating to the total weight of the tablet core.

8. The pharmaceutical composition of any of the preceding claims, the longest diameter of the tablet being below 13 mm, preferably below 12 mm.

9. The pharmaceutical composition of any of the preceding claims being in the form of an immediate release tablet.

10. The pharmaceutical composition of any of the preceding claims, comprising 100 to 400 mg, preferably about 200 mg of active ingredient calculated on the basis of its free base form.

11. A method of manufacturing the pharmaceutical composition of any of the preceding claims, wherein the active agent is blended with at least one pharmaceutically acceptable excipient and the resulting mixture is formulated into a tablet.

12. The method of claim 11, comprising a dry compaction or wet granulation step.

13. The method of claim 11, comprising a direct compression step.

14. The method of any of claims 11-13, comprising a coating step.
